(19) Europäisches Patentamt
European Patent Office
Office européen des brevets

(11) EP 3 127 943 A1

(12) **EUROPEAN PATENT APPLICATION**
published in accordance with Art. 153(4) EPC

(43) Date of publication:
**08.02.2017 Bulletin 2017/06**

(21) Application number: **15773129.0**

(22) Date of filing: **25.03.2015**

(51) Int Cl.:
***C08J 3/14*** (2006.01) ***A61K 9/14*** (2006.01)
***A61K 47/30*** (2006.01) ***A61K 49/00*** (2006.01)
***B82Y 5/00*** (2011.01) ***B82Y 40/00*** (2011.01)
***B01J 19/00*** (2006.01)

(86) International application number:
**PCT/JP2015/059077**

(87) International publication number:
**WO 2015/151952 (08.10.2015 Gazette 2015/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **31.03.2014 JP 2014073069**

(71) Applicant: **Shimadzu Corporation**
**Kyoto-shi, Kyoto 604-8511 (JP)**

(72) Inventors:
- **KAWABE, Takashi**
  **Kyoto-shi**
  **Kyoto 604-8511 (JP)**
- **OZEKI, Eiichi**
  **Kyoto-shi**
  **Kyoto 604-8511 (JP)**

(74) Representative: **Müller-Boré & Partner Patentanwälte PartG mbB**
**Friedenheimer Brücke 21**
**80639 München (DE)**

# (54) PROCESS FOR PRODUCING NANOPARTICLES

(57) Provided is a method for producing a nanoparticle having a uniform particle diameter. A method for producing a nanoparticle comprising an amphiphilic block polymer, the method comprising: with use of a nanoparticle production device that includes: a polymer solution supply channel Cp; an aqueous liquid supply channel Cw1, Cw2; a junction J of the channels; a nanoparticle formation channel Cn; and a nanoparticle-containing liquid outlet On, supplying a solution of a polymer and an aqueous liquid to the junction J; forming a nanoparticle while bringing a laminar flow of the polymer solution and a laminar flow of the aqueous liquid into contact with each other; obtaining a liquid containing the formed nanoparticle from the nanoparticle-containing liquid outlet; and controlling a particle diameter of the nanoparticle by measuring a statistic of the particle diameter of the formed nanoparticle in real time, and by controlling at least one of an amount of the polymer solution supplied to the junction and an amount of the aqueous liquid supplied to the junction such that the statistic becomes a desired value.

Fig.1

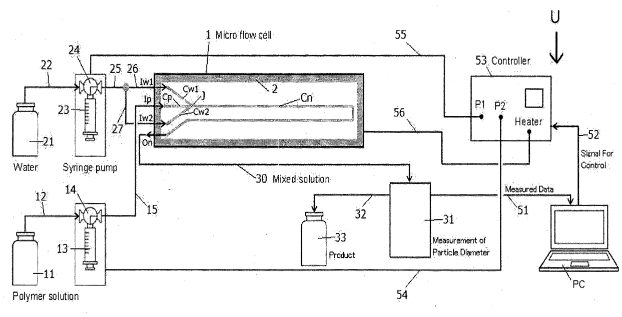


EP 3 127 943 A1

## Description

### TECHNICAL FIELD

The present invention belongs to the fields of supramolecular chemistry, collaborative region of medicine, engineering and pharmacy, and nanomedicine. The present invention relates to a method for producing a fine particle with a small particle diameter, for example, a nanoparticle with a nano-level particle diameter for use in drugs, agricultural chemicals, cosmetics, foods, and electronics (e.g., battery materials). The nanoparticle produced according to the present invention can be used as a nano-carrier for delivering various substances.

More specifically, the present invention relates to a method for producing a nanoparticle comprising an amphiphilic block polymer, and relates to a method for continuously producing a nanoparticle with a particle size controlled depending on their use and purpose.

### BACKGROUND ART

Molecular imaging techniques have been developed to diagnose tumors and other diseases. Examples of such molecular imaging techniques include positron emission tomography (PET), single photon emission computed tomography (SPECT), magnetic resonance imaging (MRI), fluorescent imaging, and the like. There has been a demand for less invasive probes suitable for these various diagnoses and having high sensitivity, that is, high accumulation selectivity to lesion sites.

When attention is paid to treatment, chemotherapy using anticancer drugs is performed in cancer treatment for preventing metastases after early treatment and after surgical treatment or radiation treatment. Anticancer drugs significantly affect also normal tissues, and therefore cause a problem of side effects. Various drug delivery systems (DDSs) have been researched and developed to reduce side effects. As one of such drug delivery systems, a DDS anticancer drug has been developed and received attention, which uses nanoparticles as a carrier and utilizes the characteristics of neovascular vessels prominently observed in the initial phase of cancer growth (Enhanced Permeability and Retention (EPR) Effect). Various nanoparticles have been studied. The EPR effect is a phenomenon in which when administered to blood, nanoparticles with a particle diameter of several tens of nanometers to several hundreds of nanometers (e.g., about 20 to 200 nm) leak from a capillary vessel system having abnormally enhanced permeability and accumulate, also due to undeveloped lymph vessels, in the interstitial space of tumor tissue that rapidly grows.

JP-A-11-335267, JP-A-2003-26812, and JP-A-2001-226294 each disclose a method for producing polymeric micelles containing a poorly water-soluble drug. JP-A-2001-226294 discloses a method for producing polymeric micelles encapsulating a poorly water-soluble drug, the method comprising: dissolving a poorly water-soluble drug and a block copolymer having a hydrophilic segment and a hydrophobic segment in a water non-miscible organic solvent to prepare an organic solution; mixing the resulting organic solution with an aqueous medium to form an oil-in-water (O/W) type emulsion; removing the organic solvent from the resulting emulsion by evaporation to form a solution of polymeric micelles encapsulating the drug; and, if necessary, subjecting the resulting solution of polymeric micelles to ultrasonic treatment and ultrafiltration treatment. However, this method disclosed in JP-A-2001-226294 comprises complicated steps because the organic solvent is removed by dialysis treatment and then the particles are fragmented by ultrasonic treatment (so-called top-down system). When the particles are fragmented by ultrasonic treatment, it is difficult to precisely control the size of the particles. Further, there is a fear that the particles once produced are broken by ultrasonic treatment so that the drug encapsulated in the particles leaks out, which makes it difficult to control the amount of the drug encapsulated in the particles.

JP-A-2012-213747 discloses a device for producing fine particles, the device comprising: a channel member having a microchannel with a diameter of 50 to 300 μm; a pressure part that applies pressure to a raw material liquid containing particles dispersed or dissolved therein to pressure-feed the raw material liquid to the channel member; and a vacuum drying chamber into which the raw material liquid that has passed through the channel member is introduced to be dried. The fine particle production device disclosed in JP-A-2012-213747 is a device in which particles that have already been formed are fragmented by pressure feeding (so-called top-down system).

JP-A-2005-246227 discloses a method for producing bubble-containing microcapsules that encapsulate a leuco dye and can be ruptured by ultrasound or pressure. The produced microcapsules have a large particle diameter of micrometer scale (for example, 4 μm in [0021] in Embodiment 1, 6 μm in [0033] in Embodiment 3). This method uses a homogenizer to fragment particles having a large particle diameter to micrometer-size particles (so-called top-down system), which makes it difficult to control the amount of a leuco dye encapsulated in the microcapsules.

JP-A-2009-256324 discloses a method for producing physiological active substance-containing fine particles, the method comprising the steps of: mixing a physiological active substance-containing aqueous volatile organic acid solution or a volatile organic acid solution of a physiological active substance with a biodegradable polymer-containing volatile organic solvent to prepare an emulsion; and mixing the resulting emulsion with an aqueous solution of a polymer

having a negative charge. However, there is no description about the particle diameter of the fine particles.

JP-A-2012-170861 discloses a method for synthesizing aspherical hydrogel particles, the method comprising: continuously introducing, into a channel structure X having at least two inlets I1 to In (n ≥ 2), at least one outlet O, and inlet channels C1 to Cn connected to the inlets I1 to In, respectively, and a channel portion J formed by joining together the inlet channels C1 to Cn simultaneously or stepwise and connected to the outlet O, a sol solution Z as a raw material of a hydrogel from the inlet I1 and a gelling agent solution G from the inlet I2 to form a fiber by gelling at least part of the sol solution Z in the channel portion J; and confining the fiber formed by gelling at least part of the sol solution Z in droplets inside or outside the channel X to cut the fiber, thereby producing aspheric hydrogel particles. According to JP-A-2012-170861, the sol solution Z is continuously introduced together with the gelling agent solution G to form a fiber by gelling the sol solution Z in the channel portion J after joining. The fiber formed by gelation is confined in droplets to cut the fiber, thereby producing aspheric hydrogel particles. JP-A-2012-170861 does not disclose an amphiphilic block polymer, and the produced hydrogel particles have a very large diameter of about several micrometers to several centimeters ([0021]).

As a nanoparticle for use in a drug delivery system (DDS), for example, JP-A-2008-24816 (US 2008/0019908) discloses an amphiphilic block polymer comprising a hydrophilic block and a hydrophobic block, wherein the hydrophilic block is a hydrophilic polypeptide chain having 10 or more sarcosine units, and the hydrophobic block is a hydrophobic molecular chain comprising units selected from the group consisting of amino acid units and hydroxyl acid units as essential structural units, and having 5 or more of the essential structural units. JP-A-2008-24816 discloses a molecular assembly comprising the amphiphilic block polymer and having a particle diameter of 10 to 500 nm.

WO 2009/148121 and Biomaterials, 2009, Vol. 30, p. 5156-5160 disclose that a linear-type amphiphilic block polymer having a polylactic acid chain as a hydrophobic block and a polysarcosine chain as a hydrophilic block self-assembles in an aqueous solution to form a polymeric micelle (lactosome) having a particle diameter of 30 nm or more. It is known that the lactosomes exhibit high retentivity in blood and the amount of the lactosomes accumulated in the liver is significantly reduced as compared to polymeric micelles that have been already developed. The lactosomes utilize the property that nanoparticles with a particle diameter of several tens of nanometers to several hundreds of nanometers retained in blood are likely to be accumulated in cancer (EPR effect), and therefore can be used as nano-carriers for cancer site-targeting molecular imaging or drug delivery.

WO 2012/176885 discloses that a branched-type amphiphilic block polymer comprising a branched hydrophilic block comprising sarcosine and a hydrophobic block comprising polylactic acid self-assembles in an aqueous solution to form a polymeric micelle (lactosome) having a particle diameter of 10 to 50 nm.

## PRIOR ART DOCUMENTS

### PATENT DOCUMENTS

Patent Document 1: JP-A-11-335267
Patent Document 2: JP-A-2003-26812
Patent Document 3: JP-A-2001-226294
Patent Document 4: JP-A-2012-213747
Patent Document 5: JP-A-2005-246227
Patent Document 6: JP-A-2009-256324
Patent Document 7: JP-A-2012-170861
Patent Document 8: JP-A-2008-24816
Patent Document 9: US 2008/0019908
Patent Document 10: WO 2009/148121
Patent Document 11: WO 2012/176885

### NON-PATENT DOCUMENT

Non-Patent Document 1: Biomaterials, 2009, Vol. 30, p. 5156-5160

## SUMMARY OF THE INVENTION

### PROBLEMS TO BE SOLVED BY THE INVENTION

In the above WO 2009/148121 and WO 2012/176885, nanoparticles are formed from an amphiphilic block

polymer by a film method. The film method includes the following steps of: preparing a solution, in a container (e.g., a glass container), containing an amphiphilic block polymer in an organic solvent; removing the organic solvent from the solution to obtain a film containing the amphiphilic block polymer on an inner wall of the container; and adding water or an aqueous solution into the container and performing ultrasonic treatment to convert the film into a particulate molecular assembly, thereby obtaining a dispersion liquid of the molecular assembly. The film method may further include the step of freeze-drying the dispersion liquid of the molecular assembly. The film method is a batch method, and is therefore undesirable from the viewpoint of productivity and unsuitable for batch-to-batch particle diameter control.

Although there is no description about actual implementation of an injection method, WO 2009/148121 and WO 2012/176885 described above also disclose production of nanoparticles by an injection method ([0145] in WO 2009/148121, [0088] in WO 2012/176885). The injection method includes the following steps of: preparing a solution, in a container (e.g., a test tube), containing an amphiphilic block polymer in an organic solvent; dispersing the solution in water or an aqueous solution; and removing the organic solvent. In the injection method, the step of purification treatment may be appropriately performed before the step of removing the organic solvent. The disclosed injection method is a batch method, and is therefore undesirable from the viewpoint of productivity and unsuitable for batch-to-batch particle diameter control.

On the other hand, in order to obtain a desired EPR effect, it is desired that nanoparticles having a uniform particle diameter of, for example, about 20 to 200 nm are produced. Further, also in fields other than the field of nanomedicine, it is desired that nanoparticles having a uniform particle diameter are produced.

An object of the present invention is to provide a method for producing a nanoparticle having a uniform particle diameter and comprising an amphiphilic block polymer.

MEANS FOR SOLVING THE PROBLEMS

The present inventors have intensively studied, and as a result, have found that a nanoparticle comprising an amphiphilic block polymer and having a uniform particle diameter in the range of about 20 to 200 nm can be continuously formed by forming a laminar flow of a solution of an amphiphilic block polymer having a hydrophilic block and a hydrophobic block and a laminar flow of an aqueous liquid, joining together the laminar flow of the polymer solution and the laminar flow of the aqueous liquid to form a nanoparticle, and measuring the particle diameter of the formed nanoparticle in real time to feed-back the measured particle diameter to the conditions of the step of forming a nanoparticle. This finding has led to the completion of the present invention.

The present invention includes the following.

(1) A method for producing a nanoparticle comprising an amphiphilic block polymer having a hydrophilic block and a hydrophobic block,
the method comprising:

with use of a nanoparticle production device that includes:

a polymer solution supply channel;
an aqueous liquid supply channel;
a junction where the polymer solution supply channel and the aqueous liquid supply channel join together;
a nanoparticle formation channel disposed downstream of the junction; and
a nanoparticle-containing liquid outlet disposed on a downstream end of the nanoparticle formation channel,

supplying a solution of an amphiphilic block polymer to the junction through the polymer solution supply channel and supplying an aqueous liquid to the junction through the aqueous liquid supply channel;
forming a nanoparticle comprising the amphiphilic block polymer while bringing a laminar flow of the polymer solution and a laminar flow of the aqueous liquid into contact with each other from the junction toward the downstream side of the nanoparticle formation channel;
obtaining a liquid containing the formed nanoparticle from the nanoparticle-containing liquid outlet; and
controlling a particle diameter of the nanoparticle by measuring a statistic of the particle diameter of the formed nanoparticle in real time, and by controlling at least one of an amount of the polymer solution supplied to the junction and an amount of the aqueous liquid supplied to the junction such that the statistic becomes a desired value.

The control of at least one of the amount of the polymer solution supplied to the junction and the amount of the aqueous liquid supplied to the junction means that the ratio between the amount of the polymer solution supplied

and the amount of the aqueous liquid supplied is varied, and/or that the total flow rate of the polymer solution supplied and the aqueous liquid supplied is varied.
(2) The method for producing a nanoparticle according to the above (1), wherein in advance:

the amphiphilic block polymer solution having a predetermined concentration is prepared, and
with use of the nanoparticle production device,
a nanoparticle comprising the amphiphilic block polymer is formed while varying at least one of the amount of the polymer solution supplied to the junction and the amount of the aqueous liquid supplied to the junction under each condition of the varied amount of the polymer solution supplied and the amount of the aqueous liquid supplied;
a statistic of the particle diameter of the nanoparticle formed under each condition is measured; and
a relationship of the amount of the polymer solution supplied and the amount of the aqueous liquid supplied with the statistic of the particle diameter of the nanoparticle is previously determined; and

at least one of the amount of the polymer solution supplied to the junction and the amount of the aqueous liquid supplied to the junction is determined and controlled, based on the relationship, to thereby control the particle size of the nanoparticle.
(3) The method for producing a nanoparticle according to the above (2), wherein a concentration of the amphiphilic block polymer solution is varied to previously determine the relationship of the amount of the polymer solution supplied and the amount of the aqueous liquid supplied with the statistic of the particle diameter of the nanoparticle.
(4) The method for producing a nanoparticle according to any one of the above (1) to (3), wherein the particle diameter is measured by a static light scattering method, a dynamic light scattering method, or a multi-angle laser light scattering method.
(5) The method for producing a nanoparticle according to any one of the above (1) to (4), wherein the amphiphilic block polymer has a hydrophilic block having an alkylene oxide unit and/or a sarcosine unit, and a hydrophobic block having a hydroxy acid unit.
(6) The method for producing a nanoparticle according to any one of the above (1) to (5), wherein the amphiphilic block polymer has a hydrophilic block having a sarcosine unit, and a hydrophobic block having a lactic acid unit.
(7) The method for producing a nanoparticle according to the above (6), wherein the hydrophilic block comprises 2 to 300 sarcosine units in total.
(8) The method for producing a nanoparticle according to the above (6) or (7), wherein the hydrophobic block comprises 5 to 400 lactic acid units.
(9) The method for producing a nanoparticle according to any one of the above (1) to (8), wherein the formed nanoparticle has a particle diameter of 10 to 200 nm.
(10) The method for producing a nanoparticle according to any one of the above (1) to (9), wherein the formed nanoparticle has a unimodal particle size distribution.
(11) The method for producing a nanoparticle according to any one of the above (1) to (10), wherein the polymer solution contains a drug and/or a labeling agent to obtain a nanoparticle containing the drug and/or the labeling agent.
(12) The method for producing a nanoparticle according to any one of the above (1) to (11), wherein the aqueous liquid contains a drug and/or a labeling agent to obtain a nanoparticle containing the drug and/or the labeling agent.
(13) A device for producing a nanoparticle comprising an amphiphilic block polymer having a hydrophilic block and a hydrophobic block, the device comprising:

a polymer solution supply channel;
an aqueous liquid supply channel;
a junction where the polymer solution supply channel and the aqueous liquid supply channel join together;
a nanoparticle formation channel disposed downstream of the junction;
a nanoparticle-containing liquid outlet disposed on a downstream end of the nanoparticle formation channel;
a particle size measurement device that measures, in real time, a statistic of a particle diameter of a nanoparticle contained in a nanoparticle-containing liquid discharged from the nanoparticle-containing liquid outlet; and
a control unit that automatically controls at least one of an amount of the polymer solution supplied and an amount of the aqueous liquid supplied based on the measured statistic of the particle diameter.

- A method for controlling a particle diameter of a nanoparticle comprising an amphiphilic block polymer having a hydrophilic block and a hydrophobic block,
  the method comprising:

with use of a nanoparticle production device that includes:

- a polymer solution supply channel;
- an aqueous liquid supply channel;
- a junction where the polymer solution supply channel and the aqueous liquid supply channel join together;
- a nanoparticle formation channel disposed downstream of the junction; and
- a nanoparticle-containing liquid outlet disposed on a downstream end of the nanoparticle formation channel,

supplying an solution of an amphiphilic block polymer to the junction through the polymer solution supply channel and supplying an aqueous liquid to the junction through the aqueous liquid supply channel;
forming a nanoparticle comprising the amphiphilic block polymer while bringing a laminar flow of the polymer solution and a laminar flow of the aqueous liquid flow into contact with each other from the junction toward the downstream side of the nanoparticle formation channel;
obtaining a liquid containing the formed nanoparticle from the nanoparticle-containing liquid outlet; and
controlling a particle diameter of the nanoparticle by measuring a statistic of the particle diameter of the formed nanoparticle in real time, and by controlling at least one of an amount of the polymer solution supplied to the junction and an amount of the aqueous liquid supplied to the junction such that the statistic becomes a desired value.

- The method for controlling a particle diameter of a nanoparticle according to the above, wherein in advance:

    the amphiphilic block polymer solution having a predetermined concentration is prepared, and
    with use of the nanoparticle production device,
    a nanoparticle comprising the amphiphilic block polymer is formed while varying at least one of the amount of the polymer solution supplied to the junction and the amount of the aqueous liquid supplied to the junction under each condition of the varied amount of the polymer solution supplied and the amount of the aqueous liquid supplied;
    a statistic of the particle diameter of the nanoparticle formed under each condition is measured; and
    a relationship of the amount of the polymer solution supplied and the amount of the aqueous liquid supplied with the statistic of the particle diameter of the nanoparticle is previously determined; and

  at least one of the amount of the polymer solution supplied to the junction and the amount of the aqueous liquid supplied to the junction is determined and controlled, based on the relationship, to thereby control the particle size of the nanoparticle.
- The method for controlling a particle diameter of a nanoparticle according to the above, wherein a concentration of the amphiphilic block polymer solution is varied to previously determine the relationship of the amount of the polymer solution supplied and the amount of the aqueous liquid supplied with the statistic of the particle diameter of the nanoparticle.
- The method for controlling a particle diameter of a nanoparticle, wherein the particle size is measured by a static light scattering method, a dynamic light scattering method, or a multi-angle laser light scattering method.

EFFECTS OF THE INVENTION

According to the present invention, it is possible to continuously and stably produce a nanoparticle comprising an amphiphilic block polymer and having a uniform particle diameter by forming a laminar flow of a solution of an amphiphilic block polymer having a hydrophilic block and a hydrophobic block, and a laminar flow of an aqueous liquid; joining together the laminar flow of the polymer solution and the laminar flow of the aqueous liquid to form a nanoparticle; and measuring a particle diameter of the formed nanoparticle in real time to feed-back the measured particle diameter to the conditions of the step of forming a nanoparticle so that the particle diameter of the nanoparticle is controlled with high reproducibility.

The nanoparticle comprising an amphiphilic block polymer produced according to the present invention have a uniform particle diameter in the range of about 20 to 200 nm (i.e., the nanoparticle has a unimodal particle size distribution), and therefore a desired EPR effect can be obtained. Therefore, molecular probes useful for a molecular imaging system can be obtained by allowing the nanoparticle to contain a labeling agent. Further, carriers useful for a drug delivery system (DDS) can be obtained by allowing the nanoparticle to contain a drug.

BRIEF DESCRIPTION OF THE DRAWINGS

[Fig. 1] Fig. 1 is a schematic view showing the structure of an example of a nanoparticle production apparatus according to the present invention.

[Fig. 2] Fig. 2 is a graph showing a change in the average particle diameter [nm] of nanoparticles (left vertical axis) and a change in PdI [-] (right vertical axis) with respect to the total flow rate [μL/min] (horizontal axis) of a polymer solution supplied and an aqueous liquid supplied.

MODE FOR CARRYING OUT THE INVENTION

[1. Amphiphilic block polymer]

An amphiphilic block polymer in the present invention is not particularly limited as long as the amphiphilic block polymer has a hydrophilic block and a hydrophobic block and can self-assemble by contact with an aqueous liquid (water or an aqueous solution) to form a nanoparticle. The nanoparticle is a nano-order size particle and includes a molecular assembly such as a micelle or a vesicle.

For example, the amphiphilic block polymer that can be used have a hydrophilic block having an alkylene oxide unit and/or a sarcosine unit and a hydrophobic block having a hydroxy acid unit. Examples of the alkylene oxide unit include an ethylene oxide unit, a propylene oxide unit, and the like. When the alkylene oxide unit is an ethylene oxide unit, the hydrophilic block contains a PEG chain. Examples of the hydroxy acid unit include glycolic acid, lactic acid, hydroxybutyric acid, and the like.

Hereinbelow, as an example of the amphiphilic block polymer, an amphiphilic block polymer will be described which has a hydrophilic block having a sarcosine unit and a hydrophobic block having a lactic acid unit. The amphiphilic block polymer may be either linear or branched. The hydrophilic block and the hydrophobic block are linked together by a linker part.

[1-1. Hydrophilic block]

In the present invention, the specific degree of the physical property, "hydrophilicity" of the hydrophilic block of the amphiphilic block polymer is not particularly limited, but, at least, the whole hydrophilic block shall be relatively more hydrophilic than a polylactic acid chain as the hydrophobic block that will be described later. Alternatively, the hydrophilic block shall be hydrophilic to such an extent that a copolymer composed of the hydrophilic block and the hydrophobic block can have amphiphilicity as a whole molecule of the copolymer. Alternatively, the hydrophilic block shall be hydrophilic to such an extent that the amphiphilic block polymer can self-assemble in a solvent to form a self-assembly, particularly, a particulate self-assembly.

In the present invention, the hydrophilic block of the amphiphilic block polymer may have a linear structure or a branched structure. When the hydrophilic block has a branched structure, each of the branches of the hydrophilic block contains sarcosine.

The kinds and ratio of structural units constituting the hydrophilic block are appropriately determined by those skilled in the art so that a resultant block can have such hydrophilicity as described above as a whole. For example, the hydrophilic block contains 2 to 300 sarcosine units in total. Specifically, when the hydrophilic block is of a linear type, the total number of sarcosine units may be, for example, about 10 to 300, 20 to 200, or 20 to 100. If the number of structural units exceeds the above range, when a molecular assembly is formed, the resultant molecular assembly tends to lack stability. If the number of structural units is less than the above range, a resultant block polymer cannot serve as an amphiphilic block polymer or formation of a molecular assembly tends to be difficult per se.

When the hydrophilic block is of a branched type, the total number of sarcosine units contained in all the branches may be, for example, 2 to 200, 2 to 100, or 2 to 10. Alternatively, the total number of sarcosine units contained in all the plurality of hydrophilic blocks may be, for example, 30 to 200 or 50 to 100. The average number of sarcosine units per one branch may be, for example, 1 to 60, 1 to 30, 1 to 10, or 1 to 6. That is, each of the hydrophilic blocks can be formed to contain sarcosine or a polysarcosine chain. If the number of structural units exceeds the above range, when a molecular assembly is formed, the resultant molecular assembly tends to lack stability. If the number of structural units is less than the above range, a resultant block polymer cannot serve as an amphiphilic block polymer or formation of a molecular assembly tends to be difficult per se.

When the hydrophilic block is of a branched type, the number of branches of the hydrophilic block shall be 2 or more, but is preferably 3 or more from the viewpoint of efficiently obtaining a particulate micelle when a molecular assembly is formed. The upper limit of the number of branches of the hydrophilic block is not particularly limited, but is,

for example, 27. Particularly, in the present invention, the number of branches of the hydrophilic block is preferably 3. The branched structure can be appropriately designed by those skilled in the art.

Sarcosine (i.e., N-methylglycine) is highly water-soluble, and polymer of sarcosine is highly flexible, because said polymer has an N-substituted amide and therefore can be more easily cis-trans isomerized as compared to a normal amide group, and steric hindrance around the $C^{\alpha}$ carbon atom is low. The use of such a structure as a constituent block is very useful in that the block can have high hydrophilicity as its basic characteristic, or both high hydrophilicity and high flexibility as its basic characteristics.

Further, the hydrophilic block preferably has a hydrophilic group (typified by, for example, a hydroxyl group) at its end (i.e., at the end opposite to the linker part).

In the polysarcosine chain, all the sarcosine units may be either continuous or discontinuous. However, it is preferred that the polypeptide chain is molecularly-designed so that the basic characteristics thereof described above are not impaired as a whole.

[1-2. Hydrophobic block]

In the present invention, the specific degree of the physical property, "hydrophobicity" of the hydrophobic block is not particularly limited, but, at least, the hydrophobic block shall be hydrophobic enough to be a region relatively more hydrophobic than the whole hydrophilic block so that a copolymer composed of the hydrophilic block and the hydrophobic block can have amphiphilicity as a whole molecule of the copolymer, or so that the amphiphilic block polymer can self-assemble in a solvent to form a self-assembly, preferably a particulate self-assembly.

The hydrophobic block present in one amphiphilic block polymer may or may not be branched. However, it is considered that when the hydrophobic block is not branched, a hydrophilic branched type shell is denser than a hydrophobic core, and therefore a stable core/shell-type molecular assembly having a smaller particle size is easily formed.

In the present invention, the hydrophobic block contains a polylactic acid (PLA) chain. The kinds and ratio of structural units constituting the hydrophobic block are appropriately determined by those skilled in the art so that a resultant block can have such hydrophobicity as described above as a whole. For example, the hydrophobic block contains 5 to 400 lactic acid units. Specifically, for example, when the hydrophobic block is not branched, the number of lactic acid units may be, for example, 5 to 100, 15 to 60, or 25 to 45. When the hydrophobic block is branched, the total number of lactic acid units contained in all the branches may be, for example, 10 to 400, preferably 20 to 200. In this case, the average number of lactic acid units per one branch is, for example, 5 to 100, preferably 10 to 100.

If the number of structural units exceeds the above range, when a molecular assembly is formed, the resultant molecular assembly tends to lack stability. If the number of structural units is less than the above range, formation of a molecular assembly tends to be difficult per se.

When the hydrophobic block is branched, the number of branches is not particularly limited, but may be, for example, equal to or less than the number of branches of the hydrophilic block from the viewpoint of efficiently obtaining a particulate micelle when a molecular assembly is formed.

Polylactic acid has the following basic characteristics.

Polylactic acid has excellent biocompatibility and stability. Therefore, a molecular assembly obtained from the amphiphilic material containing polylactic acid as a constituent block is very useful from the viewpoint of applicability to a living body, especially a human body.

Further, polylactic acid is rapidly metabolized due to its excellent biodegradability, and is therefore less likely to accumulate in tissue other than cancer tissue in a living body. Therefore, a molecular assembly obtained from the amphiphilic material containing polylactic acid as a constituent block is very useful from the viewpoint of specific accumulation in cancer tissue.

Further, polylactic acid is excellent in solubility in low-boiling point solvents. This makes it possible to avoid the use of a hazardous high-boiling point solvent when a molecular assembly is produced from the amphiphilic material containing polylactic acid as a constituent block. Therefore, such a molecular assembly is very useful from the viewpoint of safety for a living body.

In the polylactic acid (PLA) chain constituting the hydrophobic block, all the lactic acid units may be either continuous or discontinuous. However, it is preferred that the hydrophobic block is molecularly-designed so that the basic characteristics thereof described above are not impaired as a whole.

The polylactic acid (PLA) chain constituting the hydrophobic block may be either a poly-L-lactic acid (PLLA) chain comprising L-lactic acid units, or a poly-D-lactic acid (PDLA) chain comprising D-lactic acid units. Alternatively, the polylactic acid (PLA) chain may comprise both L-lactic acid units and D-lactic acid units. In this case, L-lactic acid units and D-lactic acid units may be arranged in an alternating, block, or random fashion.

[1-3. Ratio of number of sarcosine units to number of lactic acid units]

In the amphiphilic block polymer, when the number of sarcosine units (i.e., the number of sarcosine units contained in the hydrophilic block, or the total number of sarcosine units contained in all the branches when the hydrophilic block is branched) is defined as $N^S$, and the number of lactic acid units (i.e., the number of lactic acid units contained in the hydrophobic block, or the total number of lactic acid units contained in all the branches when the hydrophobic block is branched) is defined as $N^L$, the ratio of $N^S/N^L$ may be, for example, 0.05 to 5 or 0.05 to 4. More preferably, the ratio of $N^S/N^L$ may be 0.05 or more and less than 1.8, for example, 0.05 or more and 1.7 or less, 0.05 or more and 1.67 or less, 0.1 or more and 1.7 or less, or 0.1 or more and 1.67 or less.

[1-4. Polymer structure]

The structure of the linker site that links the hydrophilic block and the hydrophobic block together is not particularly limited as long as it is a chemically acceptable structure. The linker site can be appropriately molecularly-designed by those skilled in the art.

When the hydrophilic block has a branched structure, for example, when the number of branches of the hydrophilic block side is 2, two molecular chains containing a polysarcosine chain may extend as branches from one N atom present in the linker site of a molecular chain containing a polylactic acid chain. In other words, an N atom directly or indirectly bound to a polylactic acid chain may be directly or indirectly bound to two polysarcosine chains.

Further, for example, when the number of branches of the hydrophilic block side is 3, three molecular chains containing a polysarcosine chain may extend as branches from one C atom present in the linker site of a molecular chain containing a polylactic acid chain. In other words, a C atom directly or indirectly bound to a polylactic acid chain may be directly or indirectly bound to three polysarcosine chains. The same applies when branching occurs at one P or Si atom present in the linker site, or when the whole molecule of the amphiphilic block polymer forms a quaternary ammonium molecule.

When the number of branches of the hydrophilic block side exceeds 3, the hydrophilic block side can be molecularly-designed so that the branches further have a branched structure.

When the hydrophobic block side is also branched, the hydrophobic block side can be molecularly-designed from the same viewpoint as described above.

A preferred structure of the branched-type amphiphilic block polymer in which the number of branches of the hydrophilic block side is 3 and the hydrophobic block side is not branched is represented by the following formula (I).

[Chemical Formula 1]

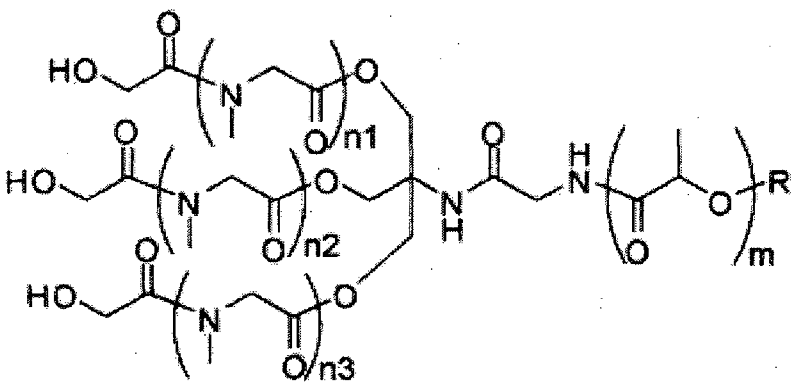

(I)

In the formula (I), n1, n2 and n3 represent numbers whose sum is 3 to 200, m represents a number of 5 to 100, and R represents a hydrogen atom or an organic group. The number of carbon atoms in the organic group may be 1 to 20. Specific examples of the organic group include alkyl groups, alkylcarbonyl groups, and the like.

A preferred structure of the branched-type amphiphilic block polymer in which the number of branches of the hydrophilic block side is 3 and the number of branches of the hydrophobic block side is 2 is represented by the following formula (II).

[Chemical Formula 2]

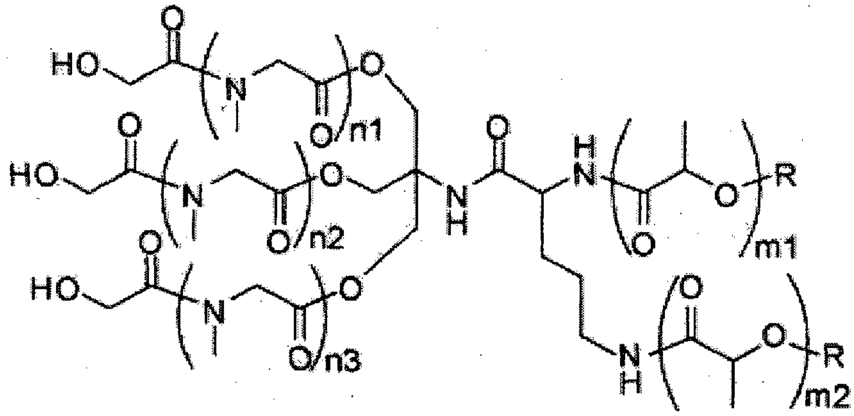

(II)

In the formula (II), n1, n2, and n3 and R are the same as those in the formula (I) and m1 and m2 represent numbers whose sum is 10 to 400.

[1-5. Synthesis method of amphiphilic block polymer>

The linear-type amphiphilic block polymer can be appropriately synthesized by those skilled in the art. For example, a hydrophobic block part (polylactic acid part) is synthesized, and a functional group (e.g., an amino group) that can be a linker to be linked to a hydrophilic block part (polysarcosine part) is introduced into one end of a polylactic acid chain. Then, polysarcosine may be introduced into this amino group.

In the synthesis of the branched-type amphiphilic block polymer, the synthesis of a hydrophobic block part (polylactic acid part), the synthesis of a hydrophilic block part (sarcosine part or polysarcosine part), and the synthesis of a linker part that links these blocks together are performed.

The branched-type amphiphilic block polymer can be synthesized by, for example, synthesizing a linker reagent that links sarcosine or polysarcosine chains and a polylactic acid chain(s) together, and then using the linker reagent as an initiator to perform attachment of a sarcosine site or extension of a polysarcosine site by polymerization reaction and extension of a polylactic acid site by polymerization reaction.

Further, the branched-type amphiphilic block polymer can be synthesized by, for example, extending a polylactic acid chain (s) after attachment of sarcosine to a linker reagent, or after attachment of polysarcosine chains, previously prepared as a hydrophilic block by polymerization reaction, to a linker reagent.

Furthermore, the branched-type amphiphilic block polymer can be synthesized by, for example, previously preparing both polysarcosine or polysarcosine chains and polylactic acid chains as a hydrophilic block and a hydrophobic block, respectively, and linking these blocks together using a linker reagent separately synthesized.

The linker reagent can have a structure in which the number of functional groups (e.g., hydroxyl groups, amino groups or the like) that can be bound to a lactic acid monomer (lactic acid or lactide) or a polylactic acid chain is one or equal to the desired number of branches of the hydrophobic block side and the number of functional groups (e.g., amino groups) that can be bound to a sarcosine monomer (e.g., sarcosine or N-carboxysarcosine anhydride) or polysarcosine is equal to the desired number of branches of the hydrophilic block side. In this case, the linker reagent is appropriately molecularly-designed by those skilled in the art so that each of the functional groups that can be bound to a sarcosine monomer or polysarcosine has the same reactivity as much as possible.

The functional group that can be bound to a lactic acid monomer or a polylactic acid chain and the functional group that can be bound to a sarcosine monomer or polysarcosine may each be protected with a protective group. In this case, as their respective protective groups, those that can be selectively removed if necessary are appropriately selected by those skilled in the art. For example, a linker reagent used to synthesize the branched-type amphiphilic block polymer whose number of branches of the hydrophilic block side is 3 can be prepared based on, for example, a tris(hydroxymethyl)aminomethane (Tris) structure.

Further, in the case of allowing the hydrophobic block side to be branched, a linker reagent can be prepared based on, for example, a structure having a larger number of branching points than the tris(hydroxymethyl)aminomethane structure. The structure having a larger number of branching points can be obtained in the following manner: a derivative of amino acid (specific examples thereof include lysine and ornithine) having, in its side chain, an amino group as an

example of a functional group that can be bound to a polylactic acid chain, in which all the amino groups are protected, is attached to tris(hydroxymethyl)aminomethane, and then deprotection is performed. The number of branching points can be increased by further attaching such an amino acid derivative to free amino groups obtained by deprotection.

A method for synthesizing the polysarcosine chain or the polylactic acid chain can be appropriately determined by those skilled in the art depending on the kind of functional group in a linker reagent, and may be selected from known peptide synthesis methods or polyester synthesis methods.

Peptide synthesis is preferably performed by, for example, ring-opening polymerization of N-carboxysarcosine anhydride (sarcosine NCA) using, as an initiator, a basic group, such as an amino group, in a linker reagent.

Polyester synthesis is preferably performed by, for example, ring-opening polymerization of lactide using, as an initiator, a basic group, such as an amino group, in a linker reagent.

When a branched-type amphiphilic block polymer different in the number of branches from the above specific example is synthesized, the branched-type amphiphilic block polymer can be prepared by those skilled in the art with appropriately making various changes in terms of organic chemistry.

The chain length of the polysarcosine chain or the polylactic acid chain can be adjusted by adjusting a loading ratio between the initiator and the monomer in the polymerization reaction. The chain length can be also determined by, for example, $^1$HNMR.

[2. Molecular assembly]

In the present invention, a nano-ordered molecular assembly (nanoparticle) such as a micelle, a multiple micelle, or a vesicle is formed by aggregation or self-assembling orientation of the amphiphilic block polymer having a hydrophilic block and a hydrophobic block.

Hereinbelow, the molecular assembly will be described with reference to a case where, as an example of the amphiphilic block polymer, one having a hydrophilic block having a sarcosine unit and a hydrophobic block having a lactic acid unit is used.

In the present invention, the molecular assembly (lactosome) is a structure formed by aggregation or self-assembling orientation of the linear- or branched-type amphiphilic block polymer described above. The molecular assembly in the present invention is preferably a micelle constituted to have a hydrophobic block on the inside (core) and a hydrophilic block on the outside (shell) in terms of practical use. The molecular assembly according to the present invention is allowed to contain an appropriate metal ion so as to be a useful structure as a probe for molecular imaging or a preparation for a drug delivery system.

The branched-type amphiphilic block polymer is larger in the molecular cross-sectional area of a hydrophilic site than the linear amphiphilic block polymer due to the presence of a plurality of polysarcosine chains as branched chains. Therefore, the molecular assembly composed of the branched-type amphiphilic block polymer is excellent in stability as a particle. Further, such a particle can have a large curvature. Therefore, the molecular assembly composed of the branched-type block polymer has a basic characteristic that said molecular assembly can have a reduced particle size.

Further, the molecular assembly composed of the branched-type amphiphilic block polymer has a basic characteristic that said molecular assembly has a higher hydrophilic group density on the surface than the lactosome composed of the linear-type amphiphilic block polymer due to the presence of a plurality of polysarcosine chains as branched chains, and therefore a hydrophobic site in said molecular assembly is less exposed.

Examples of an organic solvent for dissolving the amphiphilic block polymer include trifluoroethanol, ethanol, hexafluoroisopropanol, dimethylsulfoxide, dimethylformamide, and the like. The aqueous liquid means water or an aqueous solution. As the aqueous liquid, distilled water, distilled water for injection, normal saline, a buffer solution, or the like is used.

[3. Formation of molecular assembly]

Production of the molecular assembly (nanoparticle) composed of the amphiphilic block polymer having a hydrophilic block and a hydrophobic block will be described with reference to the drawings.

Fig. 1 is a schematic view showing the structure of an example of a nanoparticle production apparatus according to the present invention. The nanoparticle production apparatus shown in Fig. 1 includes:

a polymer solution supply channel Cp;
aqueous liquid supply channels Cw1 and Cw2;
a junction J where the polymer solution supply channel Cp and the aqueous liquid supply channels Cw1 and Cw2 join together;
a nanoparticle formation channel Cn disposed downstream of the junction J;

a nanoparticle-containing liquid outlet On disposed on a downstream end of the nanoparticle formation channel;
a particle diameter measurement device 31 that measures, in real time, a statistic of a particle diameter of a nanoparticle contained in a nanoparticle-containing liquid discharged from the nanoparticle-containing liquid outlet On; and
a control unit U that automatically controls at least one of an amount of the polymer solution supplied and an amount of the aqueous liquid supplied based on the measured statistic of the particle diameter.

As illustrated in Fig. 1, as a constituent component of the production apparatus, a micro flow cell 1 may be used, which includes:

a polymer solution inlet Ip;
a polymer solution supply channel Cp connected to the polymer solution inlet Ip;
aqueous liquid inlets Iw1 and Iw2;
aqueous liquid supply channels Cw1 and Cw2 connected to the aqueous liquid inlets Iw1 and Iw2, respectively;
a junction J where the polymer solution supply channel Cp and the aqueous liquid supply channels Cw1 and Cw2 join together;
a nanoparticle formation channel Cn disposed downstream of the junction J; and
a nanoparticle-containing liquid outlet On disposed on a downstream end of the nanoparticle formation channel.

The micro flow cell 1 is provided with a heater 2 and a thermocouple so that temperature control can be performed. Various micro flow cells are available as the micro flow cell 1.

In Fig. 1, the production apparatus has, for the purpose of supplying a polymer solution, a polymer solution tank 11 and a syringe pump 13, and further has a three-way valve 14 connected to the tank 11 through a pipe line 12 and a pipe line 15 extending from the three-way valve 14 and connected to the polymer solution inlet Ip of the micro flow cell 1.

Further, the production apparatus has, for the purpose of supplying an aqueous liquid, an aqueous liquid tank 21 and a syringe pump 23, and further has a three-way valve 24 connected to the tank 21 through a pipe line 22, a pipe line 25 extending from the three-way valve 24, and two pipe lines 26 and 27 branched off from the pipe line 25 and extending to be connected to the aqueous liquid inlets Iw1 and Iw2 of the micro flow cell 1, respectively.

In the production apparatus, the particle diameter measurement device 31 is connected to a pipe line 30 extending from the nanoparticle-containing liquid outlet On. The particle diameter measurement device 31 may be a device based on a static light scattering method, a dynamic light scattering method, or a multi-angle laser light scattering method, but usually has a measurement flow cell so that the particle diameter of a nanoparticle is measured in real time during the passage of a nanoparticle-containing liquid through the measurement flow cell. Examples of the particle diameter measurement device utilizing the dynamic light scattering method include Malvern Zetasizer nano and the like; examples of the particle diameter measurement device utilizing the static light scattering method include SALD-7100 manufactured by SHIMADZU CORPORATION and the like; and examples of the particle diameter measurement device utilizing the multi-angle laser light scattering method include DAWN HELEOS manufactured by Wyatt Technology and the like. The production apparatus has a tank 33 into which the nanoparticle-containing liquid discharged from the particle size measurement device 31 is collected through a pipe line 32.

In the production apparatus, the control unit U has a computer PC and a controller 53. The computer PC receives particle diameter data 51 measured by the particle diameter measurement device 31 and determines and controls the amount of the polymer solution supplied to the junction J and the amount of the aqueous liquid supplied to the junction J based on a previously-determined relationship of the amount of the polymer solution supplied and the amount of the aqueous liquid supplied with the particle diameter of the nanoparticle so that a measured particle diameter becomes a desired value. The controller 53 performs temperature control 56 and 2 of the micro flow cell 1 and controls the amount P2 and 54 of the polymer solution supplied to the junction J and the amount P1 and 55 of the aqueous liquid supplied to the junction J, based on a control signal 52 outputted from the computer PC. The amount of the polymer solution supplied is adjusted by the syringe pump 13 and the three-way valve 14. The amount of the aqueous liquid supplied is adjusted by the syringe pump 23 and the three-way valve 24. In the device illustrated in Fig. 1, the aqueous liquid supplied is distributed to the two aqueous liquid supply channels Cw1 and Cw2.

Using such a production device, in advance, the relationship of the amount of the polymer solution supplied and the amount of the aqueous liquid supplied (i.e., the ratio between the amount of the polymer solution supplied and the amount of the aqueous liquid supplied, and/or, the total flow rate of the polymer solution supplied and the aqueous liquid supplied) with the particle diameter of the nanoparticle is previously determined.

A amphiphilic block polymer solution having a predetermined concentration is prepared, and a nanoparticle comprising the amphiphilic block polymer is formed, at a predetermined temperature, while varying the amount of the polymer solution supplied to the junction J and the amount of the aqueous liquid supplied to the junction J under each condition of the the varied amount of the polymer solution supplied and the amount of the aqueous liquid supplied. Then,

the particle diameter of the nanoparticle formed under each condition is measured. At this time, it is not always necessary to measure the particle diameter in real time. From the obtained data, the relationship of the amount of the polymer solution supplied and the amount of the aqueous liquid supplied with the particle diameter of the nanoparticle can be determined.

Further, the relationships of the amount of the polymer solution supplied and the amount of the aqueous liquid supplied with the particle diameter of the nanoparticle at different concentrations of the amphiphilic block polymer solution can be determined by varying the concentration of the amphiphilic block polymer solution. Further, the relationships of the amount of the polymer solution supplied and the amount of the aqueous liquid supplied with the particle diameter of the nanoparticle at different temperatures can be determined by varying the temperature of formation of a nanoparticle.

The determined relationship of the amount of the polymer solution supplied and the amount of the aqueous liquid supplied with the particle diameter of the nanoparticle is previously input into the computer PC. This allows the computer PC to determine and control the amount of the polymer solution supplied to the junction J and the amount of the aqueous liquid supplied to the junction J in actual continuous production so that a measured particle diameter becomes a desired value. Therefore, a targeted monodisperse nanoparticle having a uniform particle diameter (small PdI value) is continuously produced. It is to be noted that PdI stands for polydispersity index, and when the value of PdI is smaller, the width of particle size distribution is smaller.

In the present invention, a molecular assembly (nanoparticle) having a uniform particle diameter in the range of, for example, 10 to 200 nm can be produced. The term "particle diameter" used herein refers to a particle diameter occurring most frequently in particle size distribution, that is, a mode particle diameter. However, the nanoparticle is monodisperse, and therefore the particle diameter of the nanoparticle may be an average particle diameter. A method for measuring the size of the nanoparticle according to the present invention is not particularly limited, and is appropriately selected by those skilled in the art. Examples of such a method include an observation method with a TEM (Transmission Electron Microscope) or an AFM (Atomic Force Microscope), a static light scattering method, and a DLS (Dynamic Light Scattering) method. In the case of a DLS method, the translational diffusion coefficient of particles undergoing Brownian movement in a solution is measured. In the examples of this specification, a dynamic light scattering method is used.

The particle diameter of the nanoparticle can be controlled by varying at least one of the amount of the polymer solution supplied and the amount of the aqueous liquid supplied (i..e., the ratio between the amount of the polymer solution supplied and the amount of the aqueous liquid supplied, and/or, the total flow rate of the polymer solution supplied and the aqueous liquid supplied). As shown in the examples, the particle diameter of the nanoparticle is reduced and the value of PdI is also reduced by increasing the total flow rate of the polymer solution supplied and the aqueous liquid supplied. As for the ratio between the amount of the polymer solution supplied and the amount of the aqueous liquid supplied, the particle diameter of the nanoparticle is generally reduced and the value of PdI is also reduced by increasing the ratio of the amount of the aqueous liquid supplied.

The particle diameter of particles comprising the amphiphilic block polymer whose hydrophilic block has a branched structure is smaller than that of particles comprising the amphiphilic block polymer whose hydrophilic block has a linear structure. For example, in the case of lactosome, particles comprising the linear-type polymer can have a particle diameter of about 20 to 200 nm, preferably about 25 to 40 nm, whereas particles comprising the branched-type polymer can have a particle diameter of about 10 to 30 nm. In the case of particles comprising both the linear-type polymer and the branched-type polymer, the particle diameter thereof can be further controlled by varying the ratio between them. When the particles having a smaller particle diameter (e.g., 50 nm or less) are administered to a living body, an EPR effect is more easily obtained.

The collected nanoparticle-containing liquid 33 may be appropriately subjected to post-treatment. As the step of post-treatment, the step of removing the organic solvent is performed. Further, the step of purification treatment may be appropriately performed before the step of removing the organic solvent. Examples of the purification treatment performed include gel filtration chromatography, filtering, ultracentrifugation, and the like. In this way, a solution or dispersion liquid of a nanoparticle can be obtained.

The obtained solution or dispersion liquid of a nanoparticle may be subjected to freeze-drying treatment. A method for freeze-drying treatment is not particularly limited, and any known method can be used. For example, the solution or dispersion liquid of a nanoparticle obtained in such a manner as described above may be frozen by liquid nitrogen and sublimated under reduced pressure. In this way, a freeze-dried product of the molecular assembly is obtained. That is, the molecular assembly can be stored as a freeze-dried product. If necessary, water or an aqueous solution may be added to the freeze-dried product to obtain a dispersion liquid of the molecular assembly, and the molecular assembly can be used. The water or aqueous solution is not particularly limited, and biochemically or pharmaceutically acceptable ones may be appropriately selected by those skilled in the art. Examples thereof include distilled water for injection, normal saline, a buffer solution, and the like.

[4. Drug delivery system and molecular imaging]

In the present invention, the polymer solution used to form a nanoparticle may contain a drug and/or a labeling agent to obtain a nanoparticle containing the drug and/or the labeling agent. In this case, the drug and/or the labeling agent are/is soluble in an organic solvent. Further, the aqueous liquid used to form a nanoparticle may contain a drug and/or a labeling agent to obtain a nanoparticle containing the drug and/or the labeling agent. In this case, the drug and/or the labeling agent are/is soluble in water. The drug and/or the labeling agent may be appropriately selected by those skilled in the art depending on the purpose.

[4-1. Object to which molecular assembly is to be administered]

In the present invention, a drug delivery system and molecular imaging include administration of the above-described molecular assembly to a living body. The living body to which the molecular assembly is administered is not particularly limited, but may be a human or a non-human animal. The non-human animal is not particularly limited, and may be a mammal other than a human. Specific examples thereof include primates, gnawing mammals (e.g., mice, rats), rabbits, dogs, cats, pigs, bovines, sheep, horses, and the like.

The molecular assembly used in the method according to the present invention is excellent in specific accumulation in a vascular lesion site (e.g., a malignant tumor site, an inflammatory site, an arterial sclerosis site, an angiogenic site). The molecular assembly according to the present invention accumulates in the tissue of such a site due to EPR (enhanced permeability and retention) effect, and therefore its accumulation does not depend on the kind of tissue of a vascular lesion site. The administration target of the fluorescent probe according to the present invention is preferably a cancer. Examples of the cancer as the administration target include a wide variety of cancers such as liver cancers, pancreas cancers, lung cancers, uterine cervical cancers, breast cancers, and colon cancers.

[4-2. Administration]

A method for administration to a living body is not particularly limited, and can be appropriately determined by those skilled in the art. Therefore, the administration method may be either systemic or local. That is, the molecular probes can be administered by any one of injection (needle injection or needleless injection), oral administration, and external administration.

[4-3. Detection of molecular assembly]

In the present invention, molecular imaging includes the step of detecting signals derived from the administered molecular assembly. By detecting the administered molecular assembly, it is possible to observe the appearances of an administration target (especially, the position and size of cancer tissue, and the like) from outside the body. As a detection method, any means that can visualize the administered molecular assembly can be used. The detection means can be appropriately determined by those skilled in the art depending on the kind of labeling agent of the molecular assembly.

The time from administration to the start of detection can be appropriately determined by those skilled in the art. For example, detection may be started after a lapse of 1 to 24 hours from administration. The amount of the lactosomes accumulated in a tumor site and the amounts of the lactosomes accumulated in sites other than the tumor site or temporal changes thereof are determined by a detection means.

It is to be noted that from the viewpoint of accuracy, detection of the molecular assembly is preferably performed by measuring a living body not from one direction but from two or more directions. More specifically, a living body is preferably measured from at least three directions, more preferably from at least five directions. In the case of measurement from five directions, a living body may be measured from, for example, both right and left abdomen sides, both right and left sides of the body, and back side.

EXAMPLE

Hereinbelow, the present invention will be described in more detail with reference to example, but is not limited thereto.

[Linear-type amphiphilic block polymer ($PLLA_{30}$-$PSar_{65}$)]

In the example, a linear-type amphiphilic block polymer ($PLLA_{30}$-$PSar_{65}$) having a hydrophilic block comprising 65 sarcosine units and a hydrophobic block comprising 30 L-lactic acid units was used. The polymer was synthesized

from sarcosine-NCA (Sar-NCA) and aminated poly-L-lactic acid (a-PLLA) with the use of glycolic acid, O-(benzotriazol-1-yl)-N,N,N',N'-tetramethyluronium hexafluorophosphate (HATU), and N,N-diisopropylethylamine (DIEA) in reference to the methods described in WO 2009/148121 and WO 2012/176885.

[Chemical Formula 3]

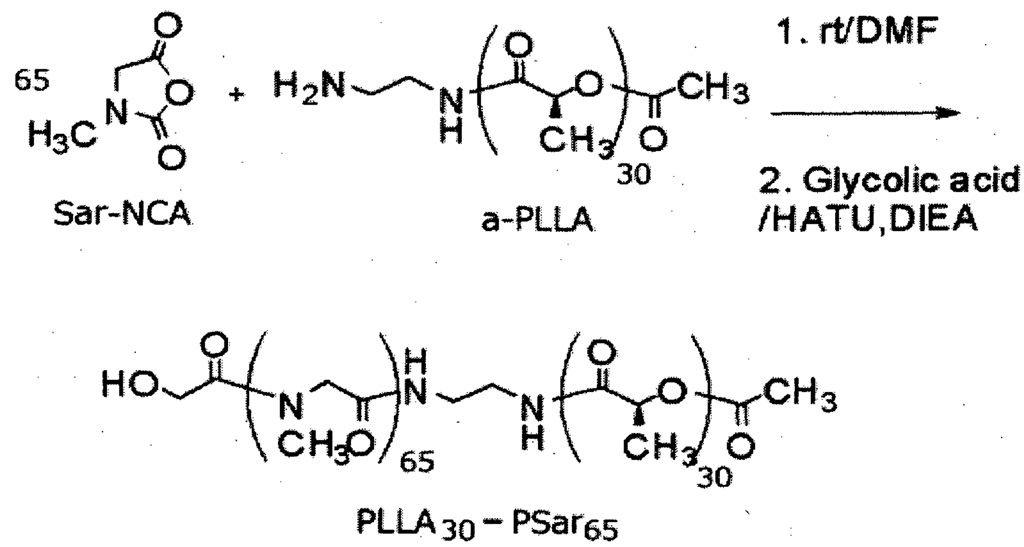


Sar-NCA

a-PLLA

$PLLA_{30}-PSar_{65}$

[Example 1]

Nanoparticles were formed from the linear-type amphiphilic block polymer ($PLLA_{30}$-$PSar_{65}$) while the particle size thereof was controlled in the following manner using the apparatus shown in Fig. 1.

As the micro flow cell 1 in Fig. 1, a chip-type mixer (Micromixer Chip Part No. 3000144 manufactured by Doromite, England) was used. As the particle diameter measurement device 31, Malvern Zetasizer nano utilizing a dynamic light scattering method was used. As the water 21, Milli-Q was used. The amphiphilic block polymer ($PLLA_{30}$-$PSar_{65}$) was dissolved in N,N-dimethylformamide (DMF) at a concentration of 10 mg/mL, and the resulting solution was stirred at 60°C for 30 minutes in an oil bath and then cooled to room temperature. The thus obtained solution was used as the polymer solution 11.

First, the computer PC sent the signal 52 to the controller 53, and the controller 53 adjusted the three-way valves 14 and 24, the syringe pumps 13 and 23, and the heater 2 based on the signal 52 to set the flow rates of the water 21 and the polymer solution 11 supplied and the temperature of the junction J of the polymer solution supply channel Cp and the aqueous liquid supply channels Cw1 and Cw2 and the nanoparticle formation channel Cn in the micro flow cell 1.

Then, the water 21 and the polymer solution 11 were mixed at the junction J under the set conditions so that nanoparticles were formed while the resulting mixture flowed from the junction J through the nanoparticle formation channel Cn. A nanoparticle-containing liquid discharged from the outlet On was sent through the pipe line 30 to a measurement flow cell of the particle diameter measurement device 31 to measure the particle diameter of the nanoparticles. Then, the nanoparticle-containing liquid was collected into the tank 33 through the pipe line 32. The results of the particle diameter measurement were stored in the computer PC.

The flow rates of the water 21 and the polymer solution 11 supplied and the temperature condition were again set to be different from those described above, and then the same operation as described above was performed. In this way, nanoparticles were formed under conditions of different flow rates and temperature, and the particle diameter of the nanoparticles formed under each of the conditions was measured to store information about a change in the particle diameter with respect to the set flow rates and the set temperature in the computer PC. From the thus obtained data, the dependence of the particle diameter on the flow rates of the water 21 and the polymer solution 11 and the temperature was formulated as a function, and the function was previously introduced into the computer PC.

When nanoparticles having a targeted particle diameter are practically continuously formed, the targeted particle diameter is previously input into the computer PC. The computer PC calculates, from the function, optimum flow rates of the water 21 and the polymer solution 11 supplied and an optimum temperature condition, and the particle diameter measurement apparatus 31 measures a particle diameter in real time and feeds back the measured particle diameter to the computer PC so that the particle diameter of the nanoparticles can be automatically controlled to be uniform. In

this way, a series of these processes makes it possible to efficiently perform particle diameter control with high reproducibility in continuous production of nanoparticles.

Fig. 2 shows the results of control of the average particle diameter (nm) of nanoparticles by controlling the total flow rate F of the water 21 and the polymer solution 11 (flow rate of water + raw material polymer). The nanoparticles were produced under the following conditions.

Raw material polymer: $PLLA_{30}$-$PSar_{65}$
Solvent: DMF (N,N-dimethylformamide)
Polymer concentration: 10 mg/mL
Mixing ratio: Distilled water/Raw material polymer solution = 9/1 (FRP=9/1)
Total flow rate F: 1,000 μL/minute to 4,000 μL/minute Substance encapsulated in nanoparticles: None
Temperature: 293K
Particle diameter measurement method: Dynamic light scattering method (DLS: Malvern Zetasizer-nano)

[Table 1]

| POINT | Z-Average[nm] | PdI[-] |
|---|---|---|
| A | 35.2 | 0.23 |
| B | 31.6 | 0.18 |
| C | 28.6 | 0.11 |
| D | 27.0 | 0.09 |

Fig. 2 is a graph showing a change in the average particle diameter [nm] of nanoparticles (left vertical axis) and a change in PdI [-] (right vertical axis) with respect to the total flow rate F [μL/minute] (horizontal axis) of the polymer solution supplied and the aqueous liquid supplied.

As shown in Fig. 2, the average particle diameter $y_1$ [nm] of nanoparticles was expressed as a function of the total flow rate x [μL/minute],

$$y_1 = 5E{-}07x^2 - 0.0052x + 39.932$$

Further, the PdI value $y_2$ [nm] of nanoparticles was expressed as a function of the total flow rate x [μL/minute], $y_2 = 1E{-}11x^3 - 8E{-}08x^2 + 0.0001x + 0.1854$

As can be seen from the above example, the average particle diameter and PdI value of nanoparticles vary dependently on the total flow rate F, and their relationships with the flow rate F(x) are represented by a quadratic approximation and a cubic approximation, respectively. In this way, the total flow rate F that should be set can be automatically calculated from a targeted average particle diameter by previously determining the resulting average particle diameter as a function of the total flow rate F, and this makes it possible to perform particle diameter control.

In the above example, the statistic of the particle diameter is an average particle diameter measured by DLS, but does not always need to be an average particle diameter, and may be the particle diameter of a single particle measured at a certain moment, or the peak particle diameter of a histogram representing the particle sizes of a plurality of particles. The statistic of the particle diameter can be variously changed as long as the physical size of formed particles is reflected.

REFERENCE SIGNS LIST

| | |
|---|---|
| 1: | Micro flow cell |
| 2: | Heater |
| J: | Junction |
| Cp: | Polymer solution supply channel |
| Cw1, Cw2: | Aqueous liquid supply channel |
| Cn: | Nanoparticle formation channel |

| | |
|---|---|
| On: | Nanoparticle-containing liquid outlet |
| 11: | Polymer solution tank |
| 13: | Syringe pump |
| 14: | Three-way valve |
| 21: | Aqueous liquid tank |
| 23: | Syringe pump |
| 24: | Three-way valve |
| 31: | Particle diameter measurement device |
| U: | Control unit |
| PC: | Computer |
| 53: | Controller |

## Claims

**1.** A method for producing a nanoparticle comprising an amphiphilic block polymer having a hydrophilic block and a hydrophobic block,
the method comprising:

with use of a nanoparticle production device that includes:

a polymer solution supply channel;
an aqueous liquid supply channel;
a junction where the polymer solution supply channel and the aqueous liquid supply channel join together;
a nanoparticle formation channel disposed downstream of the junction; and
a nanoparticle-containing liquid outlet disposed on a downstream end of the nanoparticle formation channel,

supplying a solution of an amphiphilic block polymer to the junction through the polymer solution supply channel and supplying an aqueous liquid to the junction through the aqueous liquid supply channel;
forming a nanoparticle comprising the amphiphilic block polymer while bringing a laminar flow of the polymer solution and a laminar flow of the aqueous liquid into contact with each other from the junction toward the downstream side of the nanoparticle formation channel;
obtaining a liquid containing the formed nanoparticle from the nanoparticle-containing liquid outlet; and
controlling a particle diameter of the nanoparticle by measuring a statistic of the particle diameter of the formed nanoparticle in real time, and by controlling at least one of an amount of the polymer solution supplied to the junction and an amount of the aqueous liquid supplied to the junction such that the statistic becomes a desired value.

**2.** The method for producing a nanoparticle according to claim 1, wherein in advance:

the amphiphilic block polymer solution having a predetermined concentration is prepared, and
with use of the nanoparticle production device,
a nanoparticle comprising the amphiphilic block polymer is formed while varying at least one of the amount of the polymer solution supplied to the junction and the amount of the aqueous liquid supplied to the junction under each condition of the varied amount of the polymer solution supplied and the amount of the aqueous liquid supplied;
a statistic of the particle diameter of the nanoparticle formed under each condition is measured; and
a relationship of the amount of the polymer solution supplied and the amount of the aqueous liquid supplied with the statistic of the particle diameter of the nanoparticle is previously determined; and

at least one of the amount of the polymer solution supplied to the junction and the amount of the aqueous liquid supplied to the junction is determined and controlled, based on the relationship, to thereby control the particle size of the nanoparticle.

**3.** The method for producing a nanoparticle according to claim 2, wherein a concentration of the amphiphilic block polymer solution is varied to previously determine the relationship of the amount of the polymer solution supplied and the amount of the aqueous liquid supplied with the statistic of the particle diameter of the nanoparticle.

**4.** The method for producing a nanoparticle according to any one of claims 1 to 3, wherein the particle diameter is measured by a static light scattering method, a dynamic light scattering method, or a multi-angle laser light scattering method.

**5.** The method for producing a nanoparticle according to any one of claims 1 to 4, wherein the amphiphilic block polymer has a hydrophilic block having an alkylene oxide unit and/or a sarcosine unit, and a hydrophobic block having a hydroxy acid unit.

**6.** The method for producing a nanoparticle according to any one of claims 1 to 5, wherein the amphiphilic block polymer has a hydrophilic block having a sarcosine unit, and a hydrophobic block having a lactic acid unit.

**7.** The method for producing a nanoparticle according to claim 6, wherein the hydrophilic block comprises 2 to 300 sarcosine units in total.

**8.** The method for producing a nanoparticle according to claim 6 or 7, wherein the hydrophobic block comprises 5 to 400 lactic acid units.

**9.** The method for producing a nanoparticle according to any one of claims 1 to 8, wherein the formed nanoparticle has a particle diameter of 10 to 200 nm.

**10.** The method for producing a nanoparticle according to any one of claims 1 to 9, wherein the formed nanoparticle has a unimodal particle size distribution.

**11.** The method for producing a nanoparticle according to any one of claims 1 to 10, wherein the polymer solution contains a drug and/or a labeling agent to obtain a nanoparticle containing the drug and/or the labeling agent.

**12.** The method for producing a nanoparticle according to any one of claims 1 to 11, wherein the aqueous liquid contains a drug and/or a labeling agent to obtain a nanoparticle containing the drug and/or the labeling agent.

**13.** A device for producing a nanoparticle comprising an amphiphilic block polymer having a hydrophilic block and a hydrophobic block, the device comprising:

a polymer solution supply channel;
an aqueous liquid supply channel;
a junction where the polymer solution supply channel and the aqueous liquid supply channel join together;
a nanoparticle formation channel disposed downstream of the junction;
a nanoparticle-containing liquid outlet disposed on a downstream end of the nanoparticle formation channel;
a particle size measurement device that measures, in real time, a statistic of a particle diameter of a nanoparticle contained in a nanoparticle-containing liquid discharged from the nanoparticle-containing liquid outlet; and
a control unit that automatically controls at least one of an amount of the polymer solution supplied and an amount of the aqueous liquid supplied based on the measured statistic of the particle diameter.

Fig.1

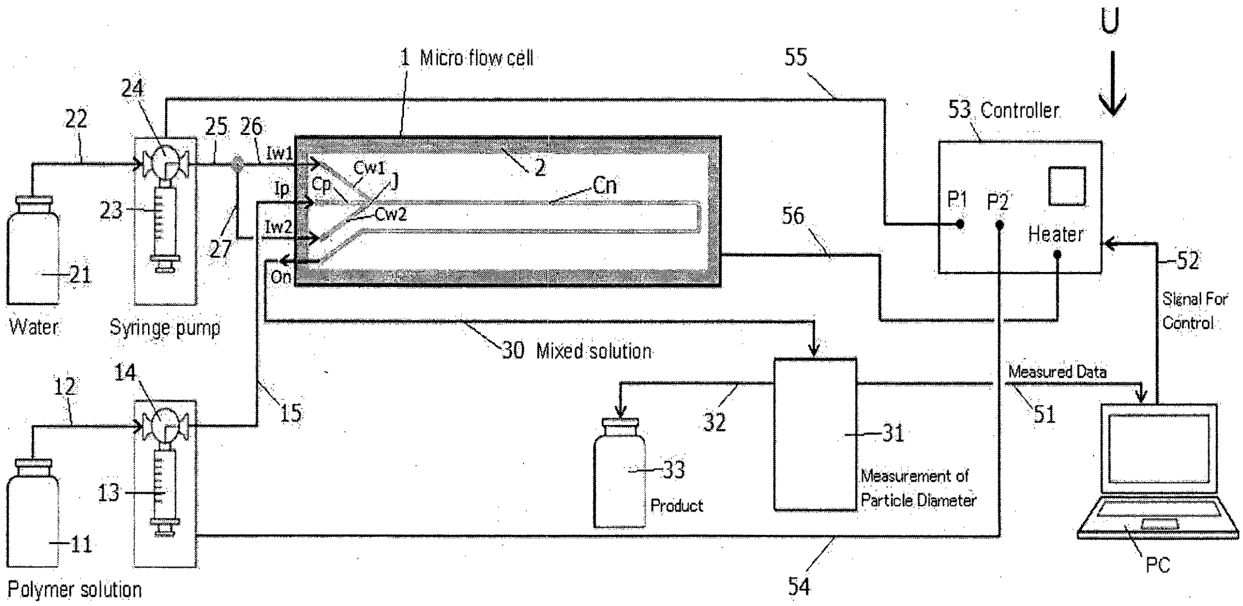
1 Micro flow cell
55
U
24
22
25 26
Iw1
Cw1
J
2
Cn
53 Controller
Ip
Cp
Cw2
P1 P2
23
Iw2
27
56
Heater
21
On
52
Water
Syringe pump
Signal For Control
30 Mixed solution
14
12
15
32
31
Measured Data
51
33
13
Product
Measurement of Particle Diameter
11
Polymer solution
54
PC

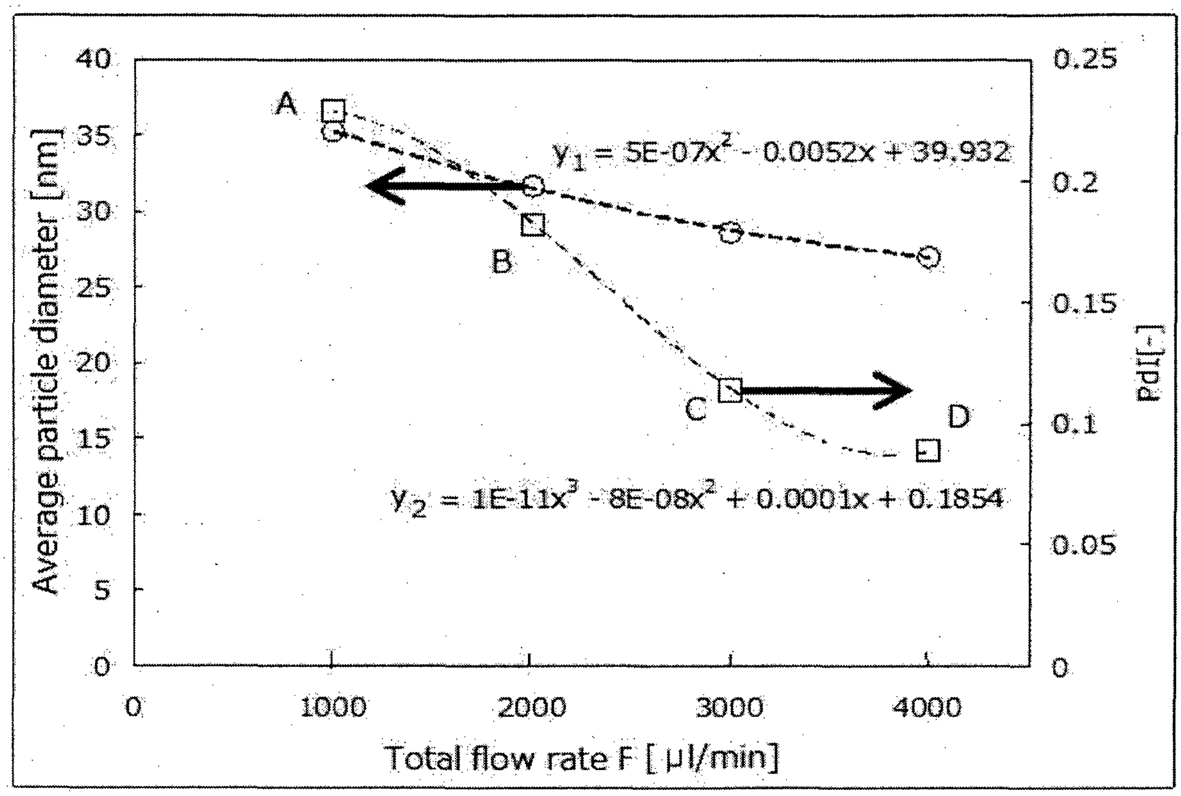

40
35
30
25
20
15
10
5
0
Average particle diameter [nm]
0.25
0.2
0.15
0.1
0.05
0
PdI[-]
A
B
C
D
y1 = 5E-07x2 - 0.0052x + 39.932
y2 = 1E-11x3 - 8E-08x2 + 0.0001x + 0.1854
0
1000
2000
3000
4000
Total flow rate F [ μl/min]


Fig.2

**INTERNATIONAL SEARCH REPORT**

International application No. PCT/JP2015/059077

A. CLASSIFICATION OF SUBJECT MATTER
*C08J3/14*(2006.01)i, *A61K9/14*(2006.01)i, *A61K47/30*(2006.01)i, *A61K49/00*(2006.01)i, *B82Y5/00*(2011.01)i, *B82Y40/00*(2011.01)i, *B01J19/00*(2006.01)n

According to International Patent Classification (IPC) or to both national classification and IPC

B. FIELDS SEARCHED

Minimum documentation searched (classification system followed by classification symbols)
C08J3/00-3/28, A61K9/00-9/72, 47/00-47/48, 49/00-49/22, B82Y5/00, 40/00, B01J19/00-19/32

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | WO 2012/176885 A1 (Shimadzu Corp. et al.), 27 December 2012 (27.12.2012), claims; paragraphs [0060] to [0064], [0081] to [0088]; examples; drawings & CN 103635514 A & EP 2725053 A1 & US 2014/0127132 A1 | 1-13 |
| Y | JP 2008-239902 A (Fujifilm Corp.), 09 October 2008 (09.10.2008), claims; paragraphs [0044], [0127]; examples; drawings (Family: none) | 1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 June 2015 (22.06.15) | 30 June 2015 (30.06.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/059077

C (Continuation). DOCUMENTS CONSIDERED TO BE RELEVANT

| Category* | Citation of document, with indication, where appropriate, of the relevant passages | Relevant to claim No. |
|---|---|---|
| P,X | JP 2014-156555 A (Shimadzu Corp.), 28 August 2014 (28.08.2014), claims; paragraphs [0103] to [0106]; examples; drawings (Family: none) | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11335267 A
- JP 2003026812 A
- JP 2001226294 A
- JP 2012213747 A
- JP 2005246227 A
- JP 2009256324 A
- JP 2012170861 A
- JP 2008024816 A
- US 20080019908 A
- WO 2009148121 A
- WO 2012176885 A


### Non-patent literature cited in the description


- *Biomaterials,* 2009, vol. 30, 5156-5160